# EUROPEAN PATENT SPECIFICATION

(11) **EP 3 342 423 B1**
(45) Date of publication and mention of the grant of the patent: **10.03.2021**
(21) Application number: 16841799.6
(22) Date of filing: 29.08.2016
(51) Int. Cl.: A61K 9/70, A61K 31/433, A61K 47/18, A61K 47/14, A61K 47/10, A61K 47/06, A61K 47/02, A61P 25/04, A61K 31/485, A61K 47/12, A61P 21/02

(54) **TRANSDERMALLY ABSORPTIVE COMPOSITION**
TRANSDERMAL ABSORBIERBARE ZUSAMMENSETZUNG
COMPOSITION D'ABSORPTION TRANSDERMIQUE

(30) Priority: 29.08.2015 WO PCT/JP2015/074553
(43) Date of publication of application: 04.07.2018
(73) Proprietor: Medrx Co., Ltd., Kagawa 769-2712 (JP)
(72) Inventor: MIWA Yasushi, Higashikagawa-shi Kagawa 769-2712 (JP); HAMAMOTO, Hidetoshi, Higashikagawa-shi Kagawa 769-2712 (JP); AKAZAWA Naoya, Higashikagawa-shi Kagawa 769-2712 (JP)
(74) Representative: Vossius & Partner Patentanwälte Rechtsanwälte mbB
(86) International application number: PCT/JP2016/075203
(87) International publication number: WO 2017/038767

(56) References cited:
- WO-A1-01/07018
- WO-A1-2009/107479
- WO-A1-2011/027786
- CN-A- 101 143 127
- JP-A- S61 172 830
- JP-A- 2007 008 871
- JP-A- 2009 529 011
- JP-A- 2012 158 521
- JP-A- 2013 528 632
- US-A1- 2004 142 024
- US-A1- 2012 226 245
- US-A1- 2013 165 522
- US-A1- 2015 174 249
- T MUNDORF ET AL: "A 12-month, multicenter, randomized, double-masked, parallel-group comparison of timolol-LA once daily and timolol maleate ophthalmic solution twice daily in the treatment of adults with glaucoma or ocular hypertension*1", CLINICAL THERAPEUTICS., vol. 26, no. 4, 1 April 2004 (2004-04-01), pages 541-551, XP55484121, US ISSN: 0149-2918, DOI: 10.1016/S0149-2918(04)90056-2

## Description

### Technical Field

The present invention relates to an external preparation comprising a basic medicament. Specifically, the present invention relates to a composition comprising sorbate as an agent for promoting the transdermal absorbability of a medicament (transdermal absorption accelerator).

### Background Art

As techniques for improving the transdermal absorbability of a basic medicament, the following two techniques have been mainly known: one is that the lipophilicity of a basic medicament is suitably adjusted by transforming the basic medicament to a salt thereof with an equimolar organic acid (Patent Document 1), and the other is that an ionic liquid made from a fatty acid and an organic amine compound is used as a transdermal absorption accelerator (Patent Document 2), both of which achieve certain results. However, the conventional techniques sometimes had some problems. For example, for some medicaments, higher skin permeability was required, or long-term stable formulations were not always prepared according to the conventional techniques.

Sorbic acid is known to be used as a preservative, for example, a preservative in a wet-type adhesive patch such as cataplasms (e.g. Patent Document 3). However, it has not been found that sorbic acid can greatly improve the transdermal absorbability of a basic medicament. Thus, it has not been aware that sorbic acid is useful as an agent for promoting the transdermal absorbability of a medicament. In addition, any non-aqueous percutaneous absorption compositions comprising sorbic acid have not been found.

Patent Document 4 discloses a pharmaceutical composition for topical application to the skin of a patient comprising a) a 1-amino-alkylcyclohexane derivative; and b) at least one gel forming agent, wherein said gel forming agent is a polymer having a neutral or positively charged polymer backbone.

Patent Document 5 relates to a transdermally absorbable preparation, having a backing and a certain adhesive layer which is placed on the backing and which contains an adhesive agent and a medicinal component.

Patent Document 6 refers to a patch formulation for external use which comprises a basic drug, an organic acid and an organic acid salt as essential components.

Patent Document 7 is directed to a phentolamine transdermal preparation for external use, which is composed of a safe and effective amount of phentolamine as a pharmaceutically active ingredient and a transdermal absorption accelerator, and pharmacologically necessary auxiliary materials, wherein the transdermal absorption enhancer is selected from natural plant volatile oil and its extracted refined products or a combined transdermal absorption enhancer with other commonly used transdermal absorption enhancers. The amount of the transdermal absorption enhancer is 0.1 to 10% w/v or 0.1 to 10% v/v.

Patent Document 8 relates to a composition for a non-aqueous patch preparation comprising a drug, an organic solvent, a lipophilic mass base, and a powder.

Non-Patent Document 1 describes a 12-month, multicenter, randomized, double-masked, parallel-group comparison of timolol-LA once daily and timolol maleate ophthalmic solution twice daily in the treatment of adults with glaucoma or ocular hypertension.

### Prior Art Documents

### Patent Documents

Patent Document 1: WO2010/016219
Patent Document 2: WO2009/066457
Patent Document 3: JP2014-125483A
Patent Document 4: US 2013/165522
Patent Document 5: US 2012/226245
Patent Document 6: US 2004/142024
Patent Document 7: CN 101143127
Patent Document 8: US 2015/174249

### Non-Patent Document

Non-Patent Document 1: T. K. Mundorf et al., Clin. Therapeutics, 26 (4), 2004, 541 - 551

### SUMMARY OF THE INVENTION

### (Problem to be Solved by the Invention)

An object of the present invention is to provide a percutaneous absorption composition having an improved transdermal absorbability of a basic medicament and an agent for promoting the transdermal absorbability of a basic medicament.

### (Means for Solving the Problem)

The present inventors have extensively studied to reach the above object, and then have found that sorbate can improve the transdermal absorbability of a basic medicament, and the improvement of the transdermal absorbability of the medicament can be further enhanced by the combination of sorbate with a basic component.

The present invention is defined in the appended claims.

### Effect of the Invention

The present invention is to provide a percutaneous absorption composition having an excellent skin permeability of a basic medicament and an agent for promoting the transdermal absorbability of a basic medicament which can produce an excellent transdermal absorption accelerating effect.

### DETAILED DESCRIPTION OF THE PREFERRED EMBODIMENTS

As defined in claim 1, a percutaneous absorption composition comprising a basic medicament or a salt thereof, and a sorbate component is provided. The sorbate component is sorbic acid and/or a metal salt thereof.

In some embodiments, the agent may further comprise a basic component.

### [Basic medicament]

According to the present invention, the basic medicament is at least one selected from the group consisting of tizanidine and oxycodone.

The active ingredient of the present invention has a basic functional group, such as an amino group. Disclosed examples thereof include a hypnotic/sedative agent such as flurazepam, rilmazafone, medetomidine, and dexmedetomidine; a stimulant/antihypnotic agent such as methamphetamine, and methylphenidate; a psychoneurotic agent such as imipramine, diazepam, sertraline, fluvoxamine, paroxetine, citalopram, fluoxetine, alprazolam, haloperidol, clomipramine, amitriptyline, desipramine, amoxapine, maprotiline, mirtazapine, setiptiline, duloxetine, diazepam, and etizolam; a local anesthetic agent such as lidocaine, procaine, tetracaine, and dibucaine; an agent for urinary organ such as oxybutynin, tamsulosin, propiverine, imidafenacin, solifenacin, darifenacin, and tolterodine; a skeletal muscle relaxant such as eperisone, pridinol, and suxamethonium; an agent for genital organ such as ritodrine and meluadrine; an autonomic agent such as carpronium, neostigmine, and bethanechol; an anti-parkinsonian agent such as pergolide, bromocriptine, trihexyphenidyl, amantadine, ropinirole, talipexole, pramipexole, rotigotine, cabergoline, selegiline, and rasagiline; an anti-migraine agent such as dihydroergotamine, sumatriptan, ergotamine, flunarizine, and cyproheptadine; an anti-histamine agent such as clemastine, diphenhydramine, chlorpheniramine, and diphenylpyraline; a bronchodilator such as tulobuterol, procaterol, salbutamol, clenbuterol, fenoterol, terbutaline, and isoprenaline; a cardiac stimulant such as denopamine; a peripheral vasodilator such as nicametate and tolazoline; a smoking-cessation aid such as nicotine and varenicline; a cardiovascular agent such as atenolol, bisoprolol, metoprolol, carvedilol, carteolol, valsartan, and clonidine; an anti-arrhythmic agent such as propranolol, alprenolol, procainamide, and mexiletine; an agent for treating peptic ulcer such as proglumide, cetraxate, spizofurone, and cimetidine; an agent for improving gastrointestinal motility such as domperidone, and cisapride; an anti-allergic agent such as ketotifen, azelastine, and emedastine; an anti-viral agent such as acyclovir; an anti-Alzheimer's agent such as donepezil, tacrine, arecoline, galantamine, and rivastigmine; a serotonin receptor antagonist/anti-emetic agent such as ondansetron, granisetron, ramosetron, and azasetron; an opioid analgesic such as morphine, codeine, fentanyl, and hydromorphone; and an anti-fungal agent such as terbinafine, butenafine, amorolfine, neticonazole, miconazole, luliconazole, and itraconazole.

The basic medicament may be used in the free form. In addition, the medicament can be used in the form of a pharmaceutically acceptable acid addition salt thereof from the viewpoint of various properties of the medicament such as its stability and low dermal irritancy. Examples of the acid addition salt include an inorganic acid salt such as hydrochloride, sulfate, and hydrobromate; and an organic acid salt such as fumarate, maleate, citrate, and tartrate.

The basic medicament or a salt thereof may be used alone or in combination with one or more other basic medicaments . In some embodiments, the basic medicament may be selected from the group consisting of tizanidine hydrochloride, oxycodone hydrochloride, optionally lidocaine hydrochloride, and a combination thereof. In some embodiments, the basic medicament may be at least one selected from the group consisting of tizanidine hydrochloride, and oxycodone hydrochloride.

### [Sorbate component]

Sorbic acid accelerates the transdermal absorbability of a basic medicament. It is thought that sorbic acid is ionically coupled to a basic medicament to form an ionic liquid in the composition, but not demonstrated. In some embodiments, the ionic liquid may be tizanidine sorbate, oxycodone sorbate or lidocaine sorbate. Both sorbic acid in the free form and a metal salt of sorbic acid such as sodium sorbate, potassium sorbate, and calcium sorbate can be used. Sorbic acid and a metal salt thereof may also be used in combination. When sorbic acid and a metal salt thereof are used in combination, the term "sorbate component" denotes a general term for sorbic acid and the metal salt thereof. In this case, the term "concentration of sorbate component" as used herein denotes the total amount of sorbic acid and a metal salt thereof. In cases where either sorbic acid or a metal salt thereof is used alone, the term "concentration of sorbate component" denotes the amount of sorbic acid or a metal salt thereof.

The concentration of sorbate component is selected from 0.5 mole to 2.5 moles, or about 0.9 mole to about 2.1 moles per mole of the basic medicament.

The transdermal absorbability of a basic medicament is markedly improved when the concentration of the sorbate component is in a range of 0.5 mole to 2.5 moles per mole of the basic medicament. When the concentration of the sorbate component exceeds 3 moles, however, the skin permeability of the basic medicament may be reduced.

### [Basic component]

In some embodiments, the composition of the present disclosure may further comprise a basic component. The basic component may include one or more organic basic compounds, inorganic basic compounds, and salts of strong bases. Inorganic basic compounds and salts of strong inorganic bases may be referred to as "inorganic basic component" as a collective term. By using the basic component in combination with the above-mentioned sorbate component, the transdermal absorbability of the basic medicament can be further improved. Thus, the combination of the above-mentioned basic component and the sorbate component can be utilized as an excellent transdermal absorption accelerator.

In some embodiments, the above-mentioned organic basic compound may be a C₂₋₉ alkanolamine such as monoethanolamine, monoisopropanolamine, diethanolamine, diisopropanolamine, triethanolamine, triisopropanolamine, ethylenediamine, and tris(hydroxymethyl)aminomethane (trometamol), or a basic amino acid such as arginine. In some embodiments, the organic basic compound is an organic amine compound having three hydroxyl groups per molecule, such as triethanolamine, triisopropanolamine, and trishydroxymethylaminomethane. In some embodiments, the organic basic compound is triethanolamine. The organic basic compound may be used alone or in combination with two or more other organic basic compounds.

In some embodiments, the above-mentioned inorganic basic component denotes an alkali metal compound or an alkali earth metal compound. Examples of the inorganic basic compound include a hydroxide such as sodium hydroxide, potassium hydroxide, and calcium hydroxide. In some embodiments, the salt of a strong base includes a metal salt of carboxylic acid such as sodium benzoate, sodium propionate, calcium propionate, sodium fumarate, sodium sorbate, and potassium sorbate; a metal salt of hydroxylic acid such as sodium lactate, sodium tartrate, potassium tartrate, and sodium citrate; sodium sulfite; and sodium pyrosulfite. In some embodiments, when the metal salt of sorbic acid such as potassium sorbate is used as the "sorbate component," it may be classified not only as a "sorbate component" but also as a "basic component". Thus, when calculating the amount of each component, the amount of the metal salt of sorbic acid is applied both as a part of the "sorbate component" and a part of the "basic component".

In some embodiments, when a basic medicament in the hydrochloride form is used as an active ingredient, and a basic component is added into the composition, the metallic ion which constitutes an inorganic basic compound and/or a salt of a strong base can act as a "hydrochloric acid scavenger". The metallic ion scavenges hydrochloric acid in the basic medicament, and thus the metallic ion in the chloride form and the basic medicament in the free form are produced. It is thought that the skin permeability of the medicament can be improved by the production of the basic medicament in the free form.

The amount of the basic component may be within the range of about 0.4 mole to about 3.0 moles, about 0.5 mole to about 2.5 moles, about 0.5 mole to about 2.0 moles, or about 0.5 mole to about 1.6 moles per mole of the sorbate component.

### [Other transdermal absorption accelerator]

In some embodiments, the composition of the present disclosure may further comprise one or more compounds that can dissolve a medicament and sorbic acid, and improve the transdermal absorbability of the medicament. For examples, an aliphatic acid, an alcohol, an ester compound, and an amide compound may be useful as a transdermal absorption accelerator. The composition of the present invention is a non-aqueous external composition which does not substantially contain water. For example, the non-aqueous composition may contain water in a concentration of less than about 3.0% or less than 1.0%.

Examples of the aliphatic acid as the transdermal absorption accelerator include one or more C₄₋₂₀ saturated or unsaturated aliphatic acids such as levulinic acid, capric acid, lauric acid, myristic acid, palmitic acid, stearic acid, isostearic acid, and oleic acid (not including sorbic acid). The aliphatic acid may be used alone or in combination with two or more aliphatic acids. In some embodiments, the composition further comprises at least one unsaturated aliphatic acid. In some embodiments, the unsaturated aliphatic acid is oleic acid. When the composition comprises aliphatic acid(s), the total amount of sorbic acid and other aliphatic acid(s) can be selected from the range of about 0.8 mole to about 2.5 moles, or about 0.8 mole to about 2.0 moles per mole of the basic component. In some embodiment, the total weight of the sorbate component and the aliphatic acid may be about 2.0% to about 3.0% by weight or about 2.0% to about 2.5% by weight relative to the total weight of the adhesive layer.

In some embodiments, the composition may further comprise an alcohol. Examples of the above-mentioned alcohol include a monovalent alcohol such as lauryl alcohol, myristyl alcohol, oleyl alcohol, isostearyl alcohol, and cetyl alcohol; a divalent alcohol such as propylene glycol, butylene glycol, dipropylene glycol, diisobutylene glycol, polyethylene glycol, and hexylene glycol; and a trivalent alcohol such as glycerin, and hexanetriol. In some embodiments, the composition may comprise two or more alcohols. In some embodiments, the composition comprises oleyl alcohol.

In some embodiments, the composition may comprise an ester compound. Examples of the ester compound include diethyl sebacate, methyl laurate, diisopropyl adipate, isopropyl myristate, propylene carbonate, and medium-chain triglyceride. Examples of the medium-chain triglyceride include triglyceride whose fatty acids have C₆₋₁₂ aliphatic chain. In some embodiments, the composition may further comprise an amide compound such as lauric acid diethanolamide.

In some embodiments, the composition can be provided as a homogeneous solution prepared by mixing a basic medicament or a salt thereof, a sorbate component, and a basic component with a solvent as described above. In some embodiments, the composition may further comprise an aliphatic acid, an alcohol, and/or an ester compound.

### [Matrix type plaster]

The composition of the present disclosure can be formulated as a matrix type plaster by dispersing the above-mentioned solution in an adhesive layer comprising an appropriate polymer. The useful polymer may include an acrylic polymer, a rubber polymer, a silicone polymer, or a vinyl ether-based polymer. In some embodiments, the rubber polymer such as styrene-isoprene-styrene block copolymer, styrene-butadiene-styrene block copolymer, polyisoprene, polyisobutylene, and polybutadiene can be suitably utilized. The concentration of the rubber polymer may be about 5% to about 40% by weight, about 10% to about 30% by weight, or about 5% to about 25% by weight relative to the total weight of the dried adhesive layer. The concentrations of the acrylic polymer and silicone polymer may be about 45 % to about 95% by weight or about 50% to about 90 % by weight relative to the total weight of the dried adhesive layer.

In some embodiments, the adhesive layer may further comprise a filler. The filler can improve not only the adhesion of the adhesive layer, but also the release rate of an active ingredient. The concentration of the filler may be selected from, for example, the range of about 0.5% to about 5% by weight relative to the total weight of the adhesive layer. The filler may be selected from hydrous silica, fumed silica, talc, crystalline cellulose, starch, carmellose, or metal salt of carmellose. In some embodiments, the filler may be fumed silica. Examples of commercially-available fumed silica include AEROSIL®.

The adhesive layer may further comprise other additive (s) such as a tackifier, a softener, and an anti-oxidant. Examples of the tackifier include rosin ester, hydrogenated rosin ester, rosin maleate, alicyclic saturated hydrocarbon resin, terpene resin, and polyolefin resin. Examples of the softener include naphthenic processing oil; vegetable oil such as camellia oil and castor oil; liquid rubber such as liquid polybutene and liquid isoprene rubber; and liquid paraffin. Examples of the anti-oxidant include dibutyl hydroxy toluene, ascorbic acid, propyl gallate, sodium sulfite, and sodium pyrosulfite.

When the matrix type plaster is prepared from the composition disclosed herein, the concentration of the basic medicament can be in the range of about 0.5% to about 10% by weight or about 1% to about 5% by weight relative to the total amount of the dried adhesive layer. Additionally, in some embodiments, at least two of a monovalent alcohol such as oleyl alcohol, a divalent alcohol such as butylene glycol, propylene glycol, and a trivalent alcohol such as glycerin are used in combination. In some embodiments, the concentration of the alcohols may be in the range of about 15% to about 35% by weight, about 20% to about 30% by weight, or about 22% to about 28% by weight relative to the total weight of the dried adhesive layer.

### EXAMPLES

Hereinafter, the present disclosure is explained in detail with examples . The present disclosure is not intended to be limited to them by any means.

### (Example 1)

### [Preparation of adhesive patch comprising tizanidine and sorbic acid]

Tizanidine hydrochloride, sorbic acid, sodium lactate, sodium benzoate, triethanolamine, diethyl sebacate, isopropyl myristate, glycerin, DiPG (dipropylene glycol), BG (butylene glycol), oleyl alcohol, propyl gallate, and liquid paraffin were mixed, then heated (at about 60°C) and dissolved to yield a homogeneous solution. Terpene resin dissolved in toluene, styrene-isoprene-styrene block copolymer (SIS5002) dissolved in toluene, and AEROSIL® were added thereto and uniformly mixed to yield a medicament-containing adhesive composition. The obtained composition was coated onto the release-treated PET film. Then, the film was heated and dried for 10 minutes at 80°C to remove toluene and prepare an adhesive patch comprising tizanidine and sorbic acid. The composition of each ingredient (% by weight) is shown in Table 1.

### (Comparative Example 1)

### [Preparation of adhesive patch comprising tizanidine and higher fatty acid, not comprising sorbic acid]

The adhesive patch with the composition (% by weight) in Table 1 was prepared in the same manner as Example 1.

### (Example 2 and Comparative Example 2)

### [Preparation of adhesive patches comprising oxycodone]

The adhesive patches comprising oxycodone with the compositions (% by weight) in Table 1 were prepared in the same manner as Example 1.

### [In vitro skin permeability test]

The skin permeability of the medicament in each adhesive patch of Examples and Comparative Examples was evaluated according to the skin permeability test using Franz cell. The accumulative skin permeation amount at each sampling point is also shown in Table 1. The skin isolated from the abdomen of a Hairless rat was used in the test on the adhesive patches comprising tizanidine prepared in Example 1 and Comparative Example 1, and the skin of a pig was used in the test on the adhesive patches comprising oxycodone prepared in Example 2 and Comparative Example 2.

**[Table 1]**

| | | Ex. 1-1 | Com. 1-1 | Com. 1-2 | Com. 1-3 | Com. 1-4 | **Ex.** 2-1 | Ex. 2-2 | Com. 2-1 | Com. 2-2 |
|---|---|---|---|---|---|---|---|---|---|---|
| | | Q907 | Q914 | Q910 | Q137 | Q891 | M304 | M305 | M300 | M292 |
| Tizanidine Hydrochloride | | 1.72 | 1.72 | 1.72 | 1.72 | 1.72 | | | | |
| Oxycodone Hydrochloride | | | | | | | 3.461 | 3.461 | 3.461 | 3.461 |
| Sorbic Acid | | 1.33 | | | | | 0.70 | 1.30 | | |
| Potassium sorbate | | | | | | | 1.30 | | | |
| Levulinic acid | | | 0.69 | 1.38 | | | | | 1.00 | |
| Isostearic acid | | | | | | 3.00 | | | | |
| Oleic acid | | | | | 4.00 | 1.00 | 3.00 | 3.00 | 4.00 | 4.00 |
| Sodium lactate | | 0.73 | 0.73 | 0.73 | 0.73 | 0.73 | | | | |
| Sodium benzoate | | 0.2 | 0.2 | 0.2 | 0.2 | 0.2 | | | | |
| Potassium hydroxide | | | | | | | | | 0.55 | |
| Triethanolamine | | 2.00 | 1.00 | 0.50 | 0.50 | 0.50 | | | | |
| Diisopropanolamine | | | | | | | 1.50 | 1.50 | 1.56 | 1.50 |
| Diethyl sebacate | | 5.00 | 5.00 | 5.00 | 5.00 | 5.00 | | | | |
| Isopropyl myristate | | 5.00 | 5.00 | 5.00 | 5.00 | 5.00 | | | | |
| Medium-chain triglyceride | | | | | | | 10.00 | 10.00 | 10.00 | 10.00 |
| Propylene carbonate | | | | | | | 5.00 | 5.00 | 5.00 | 3.00 |
| Glycerin | | 5.00 | 5.00 | 5.00 | 10.00 | 5.00 | 4.00 | 4.00 | 3.00 | 5.00 |
| Diisopropylene glycol | | 4.00 | 4.00 | 4.00 | | | | | | |
| Butylene glycol | | 3.00 | 3.00 | 3.00 | | | | | | |
| Oleyl alcohol | | 10.00 | 10.00 | 10.00 | 10.00 | 10.00 | 10.00 | 10.00 | 10.00 | 3.00 |
| PEG200 | | | | | | | | | | 7.00 |
| Benzyl alcohol | | | | | | 4.00 | | | | |
| Sodium pyrosulfite | | 0.10 | 0.10 | 0.10 | 0.10 | 0.10 | 0.10 | 0.10 | 0.10 | 0.10 |
| Propvl gallate | | 0.05 | 0.05 | 0.05 | 0.05 | 0.05 | 0.05 | 0.05 | 0.05 | 0.05 |
| AEROSIL® | | 3 | 3 | 3 | 3 | 3 | 3 | 3 | 3 | 3 |
| Liquid paraffin | | 10.87 | 12.51 | 12.32 | 11.7 | 12.7 | 16.91 | 17.61 | 17.34 | 17.89 |
| Terpene resin | | 32 | 32 | 32 | 32 | 32 | 26 | 26 | 26 | 27 |
| SIS5002 | | 16 | 16 | 16 | 16 | 16 | 15 | 15 | 15 | 15 |
| Total | | 100 | 100 | 100 | 100 | 100 | 100 | 100.02 | 100 | 100 |
| (base) / (sorbate component) | | 1.84 | | | | | 1.37 | 1.02 | | |
| (sorbate component) / (medicament) | | 2.00 | | | | | 1.75 | 1.36 | | |
| (aliphatic acid) / (base) | | 0.54 | 0.39 | 1.01 | 1.20 | 1.20 | 1.25 | 1.88 | 1.05 | 1.20 |
| Accumulative skin permeation amount (µg / cm²) | 2hr | 21.3 | 6.5 | 1.8 | 3.6 | 3.8 | 0.881 | 0.675 | | 0.235 |
| | 4hr | 80.7 | 28.2 | 10.4 | 11.9 | 15.5 | 10.39 | 11.23 | 0.491 | 8.577 |
| | 6hr | 135.3 | 49.8 | 24.9 | 24.4 | 30.6 | | | | |
| | 8hr | 172.3 | 71.5 | 40.1 | 39.8 | 46.7 | 36.12 | 73.08 | 4.059 | 44.9 |
| | 10hr | 192.8 | 87.9 | 55.8 | 56.9 | 62.4 | | | | |
| | 24hr | | | | | | 348.5 | 318.2 | 40.22 | 197.2 |

It was shown that the adhesive patch of Example 1-1 comprising sorbic acid produced a higher skin permeability of the medicament than those of Comparative Examples 1-1 to 1-4 comprising higher fatty acid instead of sorbic acid. The adhesive patches of Example 2 and Comparable Example 2 comprising oxycodone instead of tizanidine also showed similar results as those comprising tizanidine. That is, it was shown that the adhesive patches of present invention comprising sorbic acid (Examples 2-1 and 2-2) produced a higher skin permeability of the medicament than the adhesive patches comprising no sorbic acid.

### (Example 3)

### [Study of optimum amounts of sorbic acid and basic component]

The adhesive patches were prepared according to the composition (% by weight) in Table 2. The *in vitro* skin permeability test using the rat's skin was conducted on the prepared adhesive patches. The results are shown in Table 2.

**[Table 2] Examples 3-7, 3-8 and 3-9 are reference examples.**

| | | Ex. 3-1 | Ex. 3-2 | Ex. 3-3 | Ex. 3-4 | Ex. 3-5 | Ex. 3-6 | Ex. 3-7 | Ex. 3-8 | Ex. 3-9 |
|---|---|---|---|---|---|---|---|---|---|---|
| | | Q909 | Q907 | Q906 | Q900 | Q899 | Q898 | Q901 | Q897 | Q905 |
| Tizanidine Hydrochloride | | 1.72 | 1.72 | 1.72 | 1.72 | 1.72 | 1.72 | 1.72 | 1.72 | 1.72 |
| Sorbic Acid | | 0.66 | 1.33 | 1.33 | 1.33 | 1.33 | 1.33 | 2.65 | 2.65 | 3.32 |
| Sodium lactate | | 0.73 | 0.73 | 0.73 | 0.73 | 0.73 | 0.73 | 0.73 | 0.73 | 0.73 |
| Sodium benzoate | | 0.20 | 0.20 | 0.20 | 0.20 | 0.20 | 0.20 | 0.20 | 0.20 | 0.20 |
| Triethanolamine | | 1.50 | 2.00 | 1. 50 | 1.00 | 0.50 | 0.00 | 1.00 | 0.50 | 0.00 |
| Diethyl sebacate | | 5.00 | 5.00 | 5.00 | 5.00 | 5.00 | 5.00 | 5.00 | 5.00 | 5.00 |
| Isopropyl myristate | | 5.00 | 5.00 | 5.00 | 5.00 | 5.00 | 5.00 | 5.00 | 5.00 | 5.00 |
| Glycerin | | 5.00 | 5.00 | 5.00 | 5.00 | 5.00 | 5.00 | 5.00 | 5.00 | 5.00 |
| Diisopropylene glycol | | 4.00 | 4.00 | 4.00 | 4.00 | 4.00 | 4.00 | 4.00 | 4.00 | 4.00 |
| Butylene glycol | | 3.00 | 3.00 | 3.00 | 3.00 | 3.00 | 3.00 | 3.00 | 3.00 | 3.00 |
| Oleyl alcohol | | 10.00 | 10.00 | 10.00 | 10.00 | 10.00 | 10.00 | 10.00 | 10.00 | 10.00 |
| AEROSIL® | | 3.00 | 3.00 | 3.00 | 3.00 | 3.00 | 3.00 | 3.00 | 3.00 | 3.00 |
| Sodium pyrosulfite | | 0.10 | 0.10 | 0.10 | 0.10 | 0.10 | 0.10 | 0.10 | 0.10 | 0.10 |
| Propyl gallate | | 0.05 | 0.05 | 0.05 | 0.05 | 0.05 | 0.05 | 0.05 | 0.05 | 0.05 |
| Liquid paraffin | | 12.04 | 10.87 | 11.37 | 11.87 | 12.37 | 12.87 | 10.55 | 11.05 | 10.88 |
| Terpene resin | | 32.00 | 32.00 | 32.00 | 32.00 | 32.00 | 32.00 | 32.00 | 32.00 | 32.00 |
| SIS5002 | | 16.00 | 16.00 | 16.00 | 16.00 | 16.00 | 16.00 | 16.00 | 16.00 | 16.00 |
| Total | | 100.0 0 | 100.0 0 | 100.0 0 | 100.0 0 | 100.0 0 | 100.0 0 | 100.0 0 | 100.0 0 | 100.0 0 |
| (base) / (sorbate component) | | 3.05 | 1.80 | 1.51 | 1.23 | 0.95 | 0.67 | 0.62 | 0.48 | 0.27 |
| (sorbate component) / (tizanidine) | | 0.99 | 2.00 | 2.00 | 2.00 | 2.00 | 2.00 | 3.99 | 3.99 | 5.00 |
| Accumulati ve skin permeation amount (µg / cm²) | 2hr | 10.80 | 21.30 | 4.40 | 14.70 | 5.80 | 4.70 | 3.40 | 3.10 | 3.50 |
| | 4hr | 35.20 | 80.70 | 29.40 | 53.80 | 33.70 | 28.90 | 25.60 | 24.00 | 20.80 |
| | 6hr | 54.00 | 135.3 0 | 63.70 | 91.30 | 66.50 | 57.60 | 48.20 | 47.50 | 45.40 |
| | 8hr | 66.70 | 172.3 0 | 93.00 | 119.3 0 | 99.40 | 85.70 | 71.90 | 72.50 | 66.90 |
| | 10hr | 77.30 | 192.8 0 | 115.2 0 | 137.7 0 | 123.6 0 | 107.5 0 | 87.30 | 93.50 | 82.80 |

The adhesive patches of Examples 3-1 to 3-9 comprising sorbic acid produced an excellent skin permeability of the medicament. On the other hand, the adhesive patch of Example 3-1 exhibited slightly less skin permeability of the medicament because the ratio of sorbic acid to basic component was small. The adhesive patches of Examples 3-7 to 3-9 comprising excessive amounts of sorbic acid relative to tizanidine also exhibited slightly less skin permeability of the medicament.

### (Example 4)

### [Study of optimum basic component]

The adhesive patches comprising sorbic acid and a variety of basic components were prepared according to the compositions (% by weight) in Table 3. The *in vitro* skin permeability test using the rat's skin was conducted on the prepared adhesive patches. The results are shown in Table 3.

**[Table 3]**

| | Ex. 4-1 | Ex. 4-2 | Ex. 4-3 | Ex. 4-4 | Ex. 4-5 | Ex. 4-6 | Ex. 4-7 | Ex. 4-8 |
|---|---|---|---|---|---|---|---|---|
| | Q916 | Q949 | Q950 | Q162 | Q161 | Q163 | Q899 | Q946 |
| Tizanidine Hydrochloride | 1.72 | 1.72 | 1.72 | 1.72 | 1.72 | 1.72 | 1.72 | 1.72 |
| Sorbic Acid | 1.33 | 1.33 | 1.33 | 1.33 | 1.33 | 1.33 | 1.33 | 0.66 |
| Potassium sorbate | | | | | | | | 0.89 |
| Sodium lactate | 0.73 | | | | | | 0.73 | |
| Sodium benzoate | 0.20 | | | | | | 0.2 | |
| Sodium hydroxide | | | | 0.24 | 0.24 | 0.24 | 0.24 | 0.24 |
| Triethanolamine | 0.50 | | | 0.25 | 0.50 | 1.00 | 0.50 | 0.50 |
| Arginine | | | 1.03 | | | | | |
| Trometamol | 0.72 | 0.72 | | | | | | |
| Diethyl sebacate | 5.00 | 5.00 | 5.00 | 5.00 | 5.00 | 5.00 | 5.00 | 5.00 |
| Isopropyl myristate | 5.00 | 5.00 | 5.00 | 5.00 | 5.00 | 5.00 | 5.00 | 5.00 |
| Glycerin | 5.00 | 5.00 | 5.00 | 5.00 | 5.00 | 5.00 | 5.00 | 5.00 |
| Diisopropylene glycol | 4.00 | 4.00 | 4.00 | | | | 4.00 | |
| Butylene glycol | 3.00 | 3.00 | 3.00 | 4.00 | 4.00 | 4.00 | 3.00 | 4.00 |
| Oleyl alcohol | 10.00 | 10.00 | 10.00 | 10.00 | 10.00 | 10.00 | 10.00 | 10.00 |
| AEROSIL® | 3.00 | 3.00 | 3.00 | 3.00 | 3.00 | 3.00 | 3.00 | 3.00 |
| Sodium pyrosulfite | 0.10 | 0.20 | 0.20 | 0.10 | 0.20 | 0.10 | 0.10 | 0.10 |
| Propyl qallate | 0.05 | 0.05 | 0.05 | 0.05 | 0.05 | 0.05 | 0.05 | 0.05 |
| Liquid paraffin | 11.65 | 13.08 | 12.77 | 11.31 | 11.06 | 10.56 | 10.37 | 11.08 |
| Terpene resin | 32.00 | 32.00 | 32.00 | 32.00 | 32.00 | 32.00 | 32.00 | 32.00 |
| SIS5002 | 16.00 | 16.00 | 16.00 | 16.00 | 16.00 | 16.00 | 16.00 | 16.00 |
| Total | 100.00 | 100.10 | 100.10 | 95.00 | 95.10 | 95.00 | 98.24 | 95.24 |
| (base) / (sorbate component) | 1.45 | 0.50 | 0.50 | 0.65 | 0.79 | 1.07 | 1.45 | 1.29 |
| (sorbate component) / (tizanidine) | 2.00 | 2.00 | 2.00 | 2.00 | 2.00 | 2.00 | 2.00 | 1.99 |
| Accumulative skin permeation amount after 8hr (µg / cm²) | 166.50 | 137.00 | 147.00 | 114.00 | 116.00 | 125.00 | 126.00 | 135.00 |

The adhesive patches of Examples 4-1 to 4-8 comprise sorbic acid, and the content ratio of sorbic acid to the medicament and the content ratio of the basic component to sorbic acid therein are appropriate. As a result, the adhesive patches produced an excellent skin permeability of the medicament.

### (Example 5)

The adhesive patches were prepared according to the composition (% by weight) in Table 4. The *in vitro* skin permeability test using the pig's skin was conducted on the prepared adhesive patches. The results are shown in Table 4.

**[Table 4]**

| | | Ex. 5-1 | Ex. 5-2 | Ex. 5-3 | Ex. 5-4 | Ex. 5-5 | Ex. 5-6 | Ex. 5-7 |
|---|---|---|---|---|---|---|---|---|
| | | T651 | T695 | T697 | T700 | T702 | T696 | T739 |
| Tizanidine Hydrochloride | | 1.72 | 1.72 | 1.72 | 1.72 | 1.72 | 1.72 | 1.72 |
| Sorbic Acid | | 0.66 | | | | | | |
| Potassium sorbate | | 0.89 | 0.89 | 0.89 | 0.89 | 0.89 | 0.89 | 0.89 |
| Oleic acid | | | 1.50 | 1.50 | 1.50 | 1.50 | 1.50 | 1.50 |
| TEA(Triethanolamine) | | 0.5 | 0.5 | 0.4 | | | | |
| Oleyl alcohol | | 10.0 | 10.0 | 10.0 | 10.0 | 10.0 | 10.0 | 10.0 |
| Myristyl alcohol | | | 3.0 | 3.0 | | 3.0 | 3.0 | |
| Glycerin | | 5.0 | 5.0 | 5.0 | 5.0 | 5.0 | 5.0 | |
| Concentrated glycerin | | | | | | | | 5.0 |
| Purified water | | | | | | | | 0.5 |
| Propylene glycol | | 4.0 | 4.0 | 4.0 | 4.0 | 4.0 | 4.0 | 8.0 |
| Butylene glycol | | 3.0 | 3.0 | 3.0 | 3.0 | 3.0 | 3.0 | 3.0 |
| Medium-chain triglyceride | | 5.0 | | | | | | |
| AEROSIL® | | 3.0 | 3.0 | 3.0 | 3.0 | 3.0 | 3.0 | 3.0 |
| Liquid paraffin | | 18.08 | 23.24 | 23.34 | 26.74 | 23.74 | 23.74 | 22.24 |
| Terpene resin | | 32.00 | 28.00 | 28.00 | 28.00 | 28.00 | 28.00 | 28.00 |
| SIS5002 | | 16.00 | 16.00 | 16.00 | 16.00 | 16.00 | 16.00 | 16.00 |
| Sodium sulfite | | | 0.10 | 0.10 | 0.10 | 0.10 | 0.10 | 0.10 |
| Sodium pyrosulfite | | 0.10 | | | | | | |
| Propyl gallate | | 0.05 | 0.05 | 0.05 | 0.05 | 0.05 | 0.05 | 0.05 |
| Total | | 100 | 100 | 100 | 100 | 100 | 100 | 100 |
| (sorbate component) / (base) | | 1.20 | 0.59 | 0.63 | 0.88 | 0.88 | 0.88 | 0.88 |
| (base) / (sorbate component) | | 0.83 | 1.70 | 1.59 | 1.13 | 1.13 | 1.13 | 1.13 |
| (sorbate component) / (tizanidine) | | 1.99 | 1.00 | 1.00 | 1.00 | 1.00 | 1.00 | 1.00 |
| (aliphatic acid) / (base) | | 1.20 | 1.12 | 1.20 | 1.67 | 1.67 | 1.67 | 1.67 |
| Accumulative skin permeation amount (µg / cm²) | 4hr | 0.4 | 2.1 | 1.9 | 3.6 | 0.8 | 2.4 | 3.5 |
| | 6hr | 1.6 | 5.8 | 5.7 | 7.0 | 2.7 | 8.5 | 8.1 |
| | 8hr | 4.5 | 10.5 | 11.0 | 12.0 | 6.1 | 17.7 | 14.6 |
| | 22hr | 84.0 | 51.2 | 54.5 | 68.7 | 57.8 | 105.2 | 91.9 |
| | 24hr | 98.5 | 56.1 | 58.9 | 77.3 | 66.4 | 112.5 | 103.3 |

It was shown that the use of potassium sorbate as the sorbate component produced a high skin permeability of the medicament as with the use of sorbic acid.

### (Example 6)

The adhesive patches were prepared according to the composition (% by weight) in Table 5. The *in vitro* skin permeability test using pig's skin was conducted on the prepared adhesive patches. The results are shown in Table 5.

**[Table 5]**

| | | Ex. 6-1 | Ex. 6-2 | Ex. 6-3 | Ex. 6-4 | Com. 6-1 | Com. 6-2 | Com. 6-3 | Com. 6-4 |
|---|---|---|---|---|---|---|---|---|---|
| | | M304 | M306 | M305 | M307 | M291 | M295 | M300 | M303 |
| Oxyco. HCl 3H₂O | | 3.461 | 3.461 | 3.461 | 3.461 | 3.461 | 3.461 | 3.461 | 3.461 |
| Sorbic acid | | 0.7 | 0.7 | | | | | | |
| Potassium sorbate | | 1.3 | 1.3 | 1.3 | 1.3 | | | | |
| Diisopropanolamine | | 1.5 | 1.5 | 1.5 | 1.5 | 1.5 | | 1.5 | 1.5 |
| Triethanolamine | | | | | | | 2.1 | | |
| Potassium hydroxide | | | | | | 0.55 | 0.56 | 0.55 | 0.55 |
| Levulinic acid | | | | | 0.8 | 1.0 | 1.0 | 1.0 | 1.5 |
| Oleic acid | | 3.0 | 3.0 | 3.0 | 3.0 | 4.0 | 4.0 | 4.0 | 4.0 |
| Myristic acid | | | 5.0 | | | | | | |
| Oleyl alcohol | | 10.0 | 10.0 | 10.0 | 10.0 | 3.0 | 10.0 | 10.0 | 10.0 |
| PEG200 | | | | | | 5.0 | | | |
| Propylene carbonate | | 5.0 | 5.0 | 5.0 | 5.0 | 3.0 | 4.0 | 5.0 | 5.0 |
| Glycerin | | 4.0 | 4.0 | 4.0 | 4.0 | 4.0 | 3.0 | 3.0 | 3.0 |
| Medium-chain triglyceride | | 10.0 | | 10.0 | 10.0 | 10.0 | 10.0 | 10.0 | 10.0 |
| Diethyl sebacate | | | 5.0 | | | | | | |
| Liquid paraffin | | 16.91 | 16.91 | 17.61 | 16.81 | 18.34 | 17.73 | 17.34 | 16.84 |
| AEROSIL® | | 3.0 | 3.0 | 3.0 | 3.0 | 3.0 | 3.0 | 3.0 | 3.0 |
| PX-1150N | | 26.0 | 26.0 | 26.0 | 26.0 | 27.0 | 26.0 | 26.0 | 26.0 |
| SIS-5002 | | 15.0 | 15.0 | 15.0 | 15.0 | 15.0 | 15.0 | 15.0 | 15.0 |
| Sodium pyrosulfite | | 0.1 | 0.1 | 0.1 | 0.1 | 0.1 | 0.1 | 0.1 | 0.1 |
| Sodium sulfite | | | | | | | | | |
| Propyl qallate | | 0.05 | 0.05 | 0.05 | 0.05 | 0.05 | 0.05 | 0.05 | 0.05 |
| Total | | 100 | 188 | 100 | 100 | 100 | 100 | 100 | 100 |
| (base) / (sorbate component) | | 1.38 | 1.38 | 2.38 | 2.38 | | | | |
| (sorbate component) / (oxycodone) | | 1.73 | 1.73 | 1.00 | 1.00 | 0.00 | 0.00 | 0.00 | 0.00 |
| (aliphatic acid) / (base) | | 1.25 | 2.33 | 0.94 | 1.28 | 1.05 | 0.93 | 1.05 | 1.25 |
| Accumulativ e skin permeation amount (µg / cm²) | 2hr | 0.881 | 0.858 | 0.675 | | 1.000 | 1.561 | | 0.08 |
| | 4hr | 10.39 | 6.52 | 11.23 | 3.997 | 11.22 | 14.28 | 0.491 | 6.088 |
| | 6hr | 33.39 | 22.43 | 41.75 | 15.8 | 27.51 | 33.3 | 1.789 | 20.36 |
| | 8hr | 68.12 | 45.37 | 73.08 | 33.5 | 47.66 | 62.07 | 4.059 | 39.98 |
| | 24hr | 348.5 | 316.5 | 318.2 | 224.4 | 183.5 | 186.8 | 40.22 | 182.1 |

It was shown that the adhesive patches of Examples 6-1 to 6-4 comprising sorbic acid produced an excellent skin permeability of the medicament.

### Industrial Applicability

The percutaneous absorption composition of the present invention can be used as an adhesive patch comprising a basic medicament or a salt thereof, wherein the basic medicament is at least one selected from the group consisting of tizanidine and oxycodone.

## Claims

1. A percutaneous absorption composition comprising
a basic medicament or a salt thereof, wherein the basic medicament is at least one selected from the group consisting of tizanidine and oxycodone, and
a sorbate component selected from sorbic acid and/or its metal salt, wherein the concentration of the sorbate component is 0.5 moles to 2.5 moles per mole of the basic medicament.

2. The percutaneous absorption composition according to claim 1, wherein the concentration of the sorbate component is 0.9 to 2.5 moles per mole of the basic medicament.

3. The percutaneous absorption composition according to claim 1 or 2, wherein the basic medicament is at least one selected from the group consisting of tizanidine hydrochloride and oxycodone hydrochloride.

4. The percutaneous absorption composition according to any one of claims 1 to 3, further comprising a basic component, wherein if a metal salt of sorbic acid is used as the "sorbate component" and is classified not only as a "sorbate component" but also as a "basic component", when calculating the amount of each component, the amount of the metal salt of sorbic acid is applied both as a part of the "sorbate component" and a part of the "basic component".

5. The percutaneous absorption composition according to claim 4, wherein the basic component is an organic basic compound.

6. The percutaneous absorption composition according to claim 5, wherein the concentration of the basic component is 0.4 to 3.0 moles per mole of the sorbate component.

7. The percutaneous absorption composition according to claim 5 or 6, wherein the organic basic component is selected from the group consisting of monoethanolamine, monoisopropanolamine, diethanolamine, diisopropanolamine, triethanolamine, triisopropanolamine, ethylenediamine, tris(hydroxymethyl)aminomethane, and arginine.

8. The percutaneous absorption composition according to any one of claims 1 to 7, further comprising a transdermal absorption accelerator selected from the group consisting of an aliphatic acid, an alcohol, an ester compound, and an amide compound.

9. The percutaneous composition according to any one of claims 1 to 8, which contains less than 3.0 % water.

10. A matrix-type plaster comprising the percutaneous composition according to any one of claims 1 to 9 dispersed in an adhesive layer that comprises a polymer.

## Patentansprüche

1. Eine Zusammensetzung zur perkutanen Absorption, umfassend
ein basisches Medikament oder ein Salz davon, wobei das basische Medikament mindestens eines, ausgewählt aus der Gruppe bestehend aus Tizanidin und Oxycodon,
ist und
eine Sorbat-Komponente, ausgewählt aus Sorbinsäure und/oder deren Metallsalz, wobei die Konzentration der Sorbat-Komponente 0,5 Mol bis 2,5 Mol pro Mol des basischen Medikaments beträgt.

2. Die Zusammensetzung zur perkutanen Absorption nach Anspruch 1, wobei die Konzentration der Sorbat-Komponente 0,9 bis 2,5 Mol pro Mol des basischen Medikaments beträgt.

3. Die Zusammensetzung zur perkutanen Absorption nach Anspruch 1 oder 2, wobei das basische Medikament mindestens eines, ausgewählt aus der Gruppe bestehend aus Tizanidin-Hydrochlorid und Oxycodon-Hydrochlorid, ist.

4. Die Zusammensetzung zur perkutanen Absorption nach einem der Ansprüche 1 bis 3, weiter umfassend eine basische Komponente, wobei, wenn ein Metallsalz von Sorbinsäure als die "Sorbat-Komponente" verwendet wird und nicht nur als eine "Sorbat-Komponente", sondern auch als eine "basische Komponente" kategorisiert wird, wenn die Menge jeder Komponente berechnet wird, die Menge des Metallsalzes von Sorbinsäure sowohl als ein Teil der "Sorbat-Komponente" als auch als ein Teil der "basischen Komponente" Anwendung findet.

5. Die Zusammensetzung zur perkutanen Absorption nach Anspruch 4, wobei die basische Komponente eine organische basische Verbindung ist.

6. Die Zusammensetzung zur perkutanen Absorption nach Anspruch 5, wobei die Konzentration der basischen Komponente 0,4 bis 3,0 Mol pro Mol der Sorbat-Komponente beträgt.

7. Die Zusammensetzung zur perkutanen Absorption nach Anspruch 5 oder 6, wobei die organische basische Komponente ausgewählt ist aus der Gruppe bestehend aus Monoethanolamin, Monoisopropanolamin, Diethanolamin, Diisopropanolamin, Triethanolamin, Triisopropanolamin, Ethylendiamin, Tris(hydroxymethyl)aminomethan und Arginin.

8. Die Zusammensetzung zur perkutanen Absorption nach einem der Ansprüche 1 bis 7, weiter umfassend einen Beschleuniger der transdermalen Absorption, ausgewählt aus der Gruppe bestehend aus einer aliphatischen Säure, einem Alkohol, einer Esterverbindung und einer Amidverbindung.

9. Die perkutane Zusammensetzung nach einem der Ansprüche 1 bis 8, welche weniger als 3,0% Wasser enthält.

10. Ein Pflaster vom Matrix-Typ, umfassend die perkutane Zusammensetzung nach einem der Ansprüche 1 bis 9, dispergiert in einer Klebstoffschicht, die ein Polymer umfasst.

## Revendications

1. Composition d'absorption percutanée comprenant
un médicament basique ou un sel de celui-ci, lequel médicament basique est au moins l'un choisi dans le groupe constitué par la tizanidine et l'oxycodone, et
un composant sorbate choisi parmi l'acide sorbique et/ou son sel métallique,
dans laquelle la concentration du composant sorbate est de 0,5 mole à 2,5 moles par mole du médicament basique.

2. Composition d'absorption percutanée selon la revendication 1, dans laquelle la concentration du composant sorbate est de 0,9 à 2,5 moles par mole du médicament basique.

3. Composition d'absorption percutanée selon la revendication 1 ou 2, dans laquelle le médicament basique est au moins l'un choisi dans le groupe constitué par le chlorhydrate de tizanidine et le chlorhydrate d'oxycodone.

4. Composition d'absorption percutanée selon l'une quelconque des revendications 1 à 3, comprenant en outre un composant basique, dans laquelle, si un sel métallique d'acide sorbique est utilisé en tant que "composant sorbate" et est classé non seulement en tant que "composant sorbate" mais aussi en tant que "composant basique", quand la quantité de chaque composant est calculée, la quantité du sel métallique d'acide sorbique est appliquée tant en tant que partie du "composant sorbate" qu'en tant que partie du "composant basique".

5. Composition d'absorption percutanée selon la revendication 4, dans laquelle le composant basique est un composé basique organique.

6. Composition d'absorption percutanée selon la revendication 5, dans laquelle la concentration du composant basique est de 0,4 à 3,0 moles par mole du composant sorbate.

7. Composition d'absorption percutanée selon la revendication 5 ou 6, dans laquelle le composant basique organique est choisi dans le groupe constitué par la monoéthanolamine, la monoisopropanolamine, la diéthanolamine, la diisopropanolamine, la triéthanolamine, la triisopropanolamine, l'éthylènediamine, le tris(hydroxyméthyl)aminométhane, et l'arginine.

8. Composition d'absorption percutanée selon l'une quelconque des revendications 1 à 7, comprenant en outre un accélérateur d'absorption transdermique choisi dans le groupe constitué par un acide aliphatique, un alcool, un composé ester, et un composé amide.

9. Composition d'absorption percutanée selon l'une quelconque des revendications 1 à 8, qui contient moins de 3,0 % d'eau.

10. Emplâtre de type matrice comprenant la composition percutanée de l'une quelconque des revendications 1 à 9 dispersée dans une couche adhésive qui comprend un polymère.
